# EUROPEAN PATENT APPLICATION

(11) **EP 1 683 779 A1**
(43) Date of publication of application: **26.07.2006**
(21) Application number: 04818457.6
(22) Date of filing: 05.11.2004
(51) Int. Cl.: C07C 39/16, C07C 37/20, C07B 61/00

(54) **PROCESS FOR PRODUCING BISPHENOL A**

(30) Priority: 13.11.2003 JP 2003383991
(71) Applicant: MITSUBISHI CHEMICAL CORPORATION, Tokyo 108-0014 (JP)
(72) Inventor: KOGA, Yoshio, MITSUBISHI CHEMICAL CORPORATION, Ibaraki-ken (JP)
(74) Representative: Woods, Geoffrey Corlett
(86) International application number: PCT/JP2004/016406
(87) International publication number: WO 2005/047222

(57) **Abstract**

Subject:

To provide a bisphenol A production process which can inhibit deterioration of the cation exchange resin catalyst used in the reaction step to enable long-time stabilized production of bisphenol A.

Means for Implementation:

A process for producing bisphenol A by reacting the raw materials acetone and phenol in the presence of a catalyst, in which one of the following means (1)-(3) is used:
(1) In supplying to the reaction step the raw materials and the acetone recovered from the reaction step, the lower alcohol concentration in the whole amount of acetone supplied to the reaction step is adjusted to be not more than 100 ppm.
(2) In supplying to the reaction step the raw materials and those recovered from the reaction step, the lower alcohol concentration in the reaction solution discharged from the reaction step is adjusted to be not more than 30 ppm.
(3) In supplying the raw materials and the recovered acetone to the reaction step, at least the recovered acetone in the whole supply of acetone to the reaction step is refined before supplied to the reaction step to remove the lower alcohols contained as impurities. Selected Drawing: None

## Description

### TECHNICAL FIELD

The present invention relates to a process for producing bisphenol A. More particularly, it relates to a bisphenol A production process which makes it possible to inhibit deterioration of the cation exchange resin catalyst used in the reaction step to prolong the catalyst life by reducing the lower alcohols such as methanol contained as impurities in acetone which is used as one of the raw materials.

### BACKGROUND ART

Bisphenol A is usually produced by reacting a phenol and acetone in the presence of an acid catalyst such as a cation exchange resin. The obtained reaction mixture contains, beside the objective bisphenol A, the unreacted phenol, unreacted acetone, reaction-generated water and other reaction by-products such as tinting materials. Usually, a distillation method is used for separating the objective bisphenol A from the reaction mixture. According to this method, distillation is carried out in a distillation column at a temperature lower than the boiling point of the phenol, recovering the low-boiling point materials such as the unreacted acetone, reaction-generated water and part of the unreacted phenol from the column top. The concentrated mixed solution from the column bottom is cooled to crystallize bisphenol A as a crystalline adduct with the phenol, then this crystalline adduct is separated from the mother liquor containing the reaction by-products, and the phenol is removed from the crystal adduct to recover bisphenol A.

Unreacted acetone is included in the low-boiling point materials obtained from the column top. Usually, this acetone is separated and recovered by a treatment such as distillation using an acetone separating column, and returned to the reaction step along with the freshly supplied acetone (which may hereinafter be referred to as fresh acetone).

A small quantity of methanol is contained as an impurity in the fresh acetone. Since this methanol does not affect the production reaction of bisphenol A, it is accumulated in the separated and recovered unreacted acetone (which may hereinafter be referred to as recovered acetone). When this recovered acetone is re-supplied to the reaction step with fresh acetone, the methanol concentration in the reaction mixture elevates.

Strongly acidic cation exchange resins are generally used as the acid catalyst for the above reaction, but these strongly acidic cation exchange resins have the problem that they are subject to deterioration by the action of methanol contained as an impurity in the acetone supplied to the reaction step, resulting in their shortened use-life as a catalyst.

As a solution to this problem, a method has been proposed in which the methanol concentration in the acetone supplied to the reaction step is monitored, and when the methanol concentration rose above a certain prescribed level, methanol-rich acetone is properly released out of the acetone separation column from its middle portion and the supply of fresh acetone is increased correspondingly to adjust the methanol concentration in the acetone supplied to the reaction step to maintain below 10,000 ppm by weight (see, for example, Japanese Patent Application Laid-Open (KOKAI) No. 6-92889).

However, when the methanol concentration in fresh acetone is high, the methanol concentration can not be reduced as desired by the adjustment of the amount of recovered acetone, thereby allowing early deterioration of the strongly acidic cation exchange resin used as catalyst. Such methanol deterioration of the catalyst is serious particularly when an acid ion exchange resin partially neutralized with a sulfur-containing amine compound is used as catalyst. Also, it is hardly possible to prevent deterioration of the strongly acidic cation exchange resin catalyst by merely controlling the methanol concentration in the acetone supplied to the reaction step to stay at such a level as "below 10,000 ppm by weight"; it is necessary to control the methanol concentration in the feed acetone more strictly at a lower level.

There are also known the methods for controlling the methanol concentration in the raw materials supplied to the reaction step (see, for example, Japanese translation of PCT international application (KOHYO) No. 2001-503377). In these methods, however, no regard is given to re-use of the unreacted acetone included in the low-boiling point materials.

### DISCLOSURE OF THE INVENTION

### Problem to be Solved by the Invention

The present invention has been attained in view of the above circumstances, and its object is to provide a process for producing bisphenol A which enables long-time stabilized production of bisphenol A by inhibiting deterioration of the cation exchange resin catalyst used in the reaction.

### Means for Solving the Problem

As a result of the present inventor's earnest study to solve the above problem, it has been found that the above problem can be solved by reducing the concentration of methanol contained as an impurity in the acetone supplied to the reaction step below a certain specified level, and completed the present invention on the basis of the above finding.

In the first aspect of the present invention, there is provided a process for producing bisphenol A by reacting the raw materials acetone and phenol in the presence of a catalyst,
the lower alcohol concentration in the whole amount of acetone supplied to the reaction step being adjusted to be not more than 100 ppm by weight, when in supplying to the reaction step the said raw materials and the acetone recovered from the reaction step.

In the second aspect of the present invention, there is provided a process for producing bisphenol A by reacting the raw materials acetone and phenol in the presence of a catalyst,
the lower alcohol concentration in the reaction solution discharged from the reaction step being adjusted to be not more than 30 ppm by weight, when in supplying to the reaction step the said raw materials and those recovered from the reaction step.

In the third aspect of the present invention, there is provided a process for producing bisphenol A by reacting the raw materials acetone and phenol in the presence of a catalyst, at least the acetone recovered from the reaction step in the whole supply of acetone to the reaction step being refined before supplied to the reaction step to remove the lower alcohols contained as impurities, when in supplying to the reaction step the said raw materials and the acetone recovered from the reaction step.

### Effect of the Invention

According to the bisphenol A production process of the present invention, it is possible to inhibit deterioration of the ion exchange resin catalyst used in the reaction since the concentration of methanol contained as an impurity in the acetone supplied to the reaction step is reduced. The deterioration inhibitory effect is particularly salient when an acid ion exchange resin partially modified with a sulfur-containing amine compound is used as catalyst. Also, stabilized production of bisphenol A with fixed quality is enabled as the amounts of acetone and phenol present in the reaction step and their ratio are stabilized.

### Brief Description of the Drawing

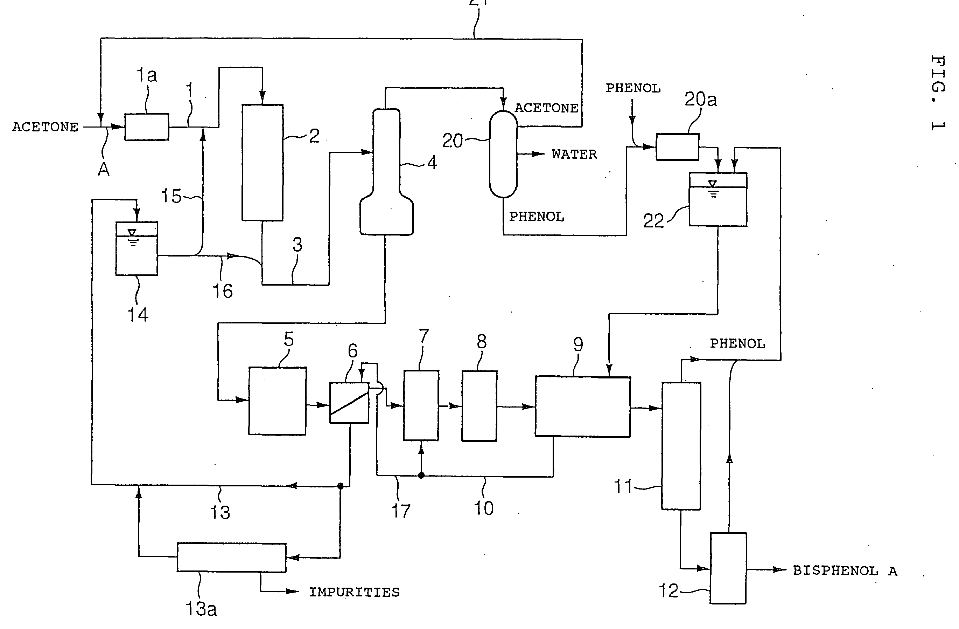

FIG. 1 is a flow chart showing the bisphenol A production process of the present invention when the method (i) was used for the separation step.

### Best Mode for Carrying out the Invention

A detailed description of the present invention is given below. In the bisphenol A production process in the first to third aspects of the present invention, the raw materials acetone and phenol are reacted in the presence of a catalyst to produce bisphenol A. The production process of the present invention usually comprises, beside the said reaction step, a separation step in which the reaction mixture obtained in the reaction step is separated into a component containing bisphenol A and a low-boiling point component containing unreacted acetone, an acetone circulating step for separating and recovering unreacted acetone from the low-boiling point component and circulating it to the reaction step, a crystallization step, a bisphenol A recovering step, and a mother liquor circulating step.

First, in order to facilitate the understanding of the present invention, the outline of the bisphenol A production process is explained with reference to FIG. 1, but the production process according to the present invention can be embodied in various ways. The flow chart of FIG. 1 shows an embodiment where the method (i) was used in the separation step described later.

Acetone and phenol, which are the raw materials, are supplied to a reactor 2 through line 1. The reaction mixture from reactor 2 is led into distillation column 4 through line 3. The low-boiling point component containing acetone, water and phenol released from the column top is forwarded to a separation system 20 and separated into acetone, water and phenol by distillation or other means. Acetone is transferred to a methanol removing device 1a as a refiner described later through line 21 and line A. Water is discharged out of the equipment from the separation system 20. Phenol is led into a refiner 20a from the separation system 20 and, after refined, transferred to a phenol storage tank 22.

The bottom products in the distillation column 4 are transferred to a crystallizer 5 where a crystalline adduct of bisphenol A and phenol is crystallized. This crystalline adduct is separated from the mother liquor by a solid/liquid separator 6, then re-dissolved by a re-dissolver 7, recrystallized by a recrystallizer 8, subjected to solid/liquid separation by a centrifuge or other means, and treated by a rinse system 9. Rinse of the crystalline adduct is conducted with purified phenol supplied from a tank 22. The rinse waste is sent to the re-dissolver 7 through line 10 (or supplied to the solid/liquid separator 6 through line 17 for reuse as rinse). After rinsed, the crystalline adduct is heated and decomposed into bisphenol A and phenol by an adduct decomposer 11, with bisphenol A being then treated by a refiner 12 to become a finished product bisphenol A. Phenol separated by the adduct decomposer 11 and refiner 12 is transferred into the tank 22.

The liquid portion (mother liquor) separated by the solid/liquid separator 6 is passed into a mother liquor tank 14 through line 13. The mother liquor in the mother liquor tank 14 is transferred to line 1 through line 15, mixed with acetone and supplied to the reactor 2. The mother liquor in the mother liquor tank 14 is also transferred to line 3 through line 16.

The supply of the mother liquor from line 15 to line 1 is controlled so that the acetone/mother liquor ratio in the acetone and mother liquor mixed solution supplied to the reactor 2 will stay within a specified range. Stabilization of the acetone/mother liquor ratio leads to a stabilized reaction in the reactor 2 and consequent stabilization of the quality and yield of the product bisphenol A. Mother liquor tank 14 can function as a buffer tank for leveling fluctuations of the circulation rate from line 13.

The steps in the production process of the present invention are explained in detail below.

### Reaction step:

In the reaction step carried out in the reactor 2, the raw materials phenol and acetone are reacted with stoichiometrically excess phenol. The phenol/acetone molar ratio is usually in the range from 3 to 30, preferably from 5 to 20. The reaction is carried out at a temperature of usually from 50 to 100°C under a pressure of usually from normal pressure to 600 kPa.

As the catalyst, usually strongly acidic cation exchange resins such as sulfonic acid type, preferably those partially neutralized with a sulfur-containing amine compound are used. As the sulfur-containing amine compound, ordinary promoters used for the synthesis of bisphenol A such as, for example, 2-(4-pyridyl)ethanethiol, 2-mercaptoethylamine, 3-mercaptopropylamine, N,N-dimethyl-3-mercaptopropylamine, N,N-di-n-butyl-4-mercaptobutylamine, and 2,2-dimethylthiazolidine can be used. Such a promoter is used in an amount of usually 2 to 30 mol%, preferably 5 to 20 mol% based on the acid group (sulfonic group) in the acid ion exchanger.

The condensation reaction of phenol and acetone is conducted according to a fixed bed flow system, which is a continuous and piston flow system, or a suspended bed batch system. In the case of the fixed bed flow system, the liquid space velocity of the mixture of the raw materials supplied to the reactor is usually 0.2 to 50 hr⁻¹. In the case of the suspended bed batch system, the amount of the strongly acid ion exchange resin used, although variable depending on the reaction temperature and pressure, is usually 20 to 100% by weight based on the mixture of the raw materials. The treatment time is usually 0.5 to 5 hours. Separating step:

The reaction mixture obtained in the reaction step is separated into a component containing bisphenol A and a low-boiling point component containing unreacted acetone. There are representatively the following three methods (i) to (iii) for separation.

### Method (i):

As explained above with reference to the flow chart of FIG. 1, the reaction mixture obtained in the reaction step is distilled in the distillation column 4, and the low-boiling point component containing unreacted acetone is separated from the column top. The bottom product is a liquid containing bisphenol A. As the distillation column, the known ones can be used. When distillation is conducted under normal pressure, the operation is performed at a temperature below the boiling point of phenol. Distillation under reduced pressure (vacuum distillation) is preferred. Vacuum distillation is usually carried out at a temperature of 50 to 150°C under a pressure of 50 to 300 mmHg. Since the unreacted phenol contained in the reaction mixture forms an adduct with bisphenol in the following crystallization step, distillation is preferably carried out under the condition that the said unreacted phenol will be discharged by a predetermined amount from the column bottom. The materials separated from the top of distillation column are unreacted acetone, water, methanol contained as an impurity, unreacted phenol, etc.

Bisphenol A concentration in the bottom product after separation of the said low-boiling point materials from the column top is usually 20 to 50% by weight. When the bisphenol A concentration is less than 20% by weight, the yield of bisphenol A decreases, and when the bisphenol A concentration exceeds 50% by weight, apparent viscosity of the concentrated mixed solution elevates to make it difficult to transfer the solution. The bottom product is transferred to the crystallization step to recover the objective bisphenol A. The crystallization step will be explained later.

### Method (ii):

The reaction mixture obtained in the reaction step is supplied directly to the crystallizer 5 (the supply line being not shown). In the crystallizer 5, the low-boiling point materials including unreacted acetone are evaporated and separated under reduced pressure to obtain a slurry of the crystalline adduct of bisphenol A and phenol. If necessary, water and acetone may be added to the reaction mixture. In the crystallization operation, the bisphenol A concentration is usually 20 to 50% by weight, the water concentration usually 1 to 10% by weight, and the acetone concentration usually 0.5 to 5% by weight.

Crystallizer 5 is depressurized to cause evaporation of water, acetone and a small quantity of phenol. Also, the crystallization material (reaction mixture) of 70 to 140°C is cooled down to usually 35 to 60°C, and a crystalline adduct is formed and reduced into a slurry. Depressurization to 50 to 550 mmHg is preferred. The obtained slurry is separated into solid and liquid by a solid/liquid separator 6 comprising a filter or other means. On the other hand, the low-boiling point materials, viz. water, acetone and accompanying phenol evaporated in the crystallizer 5 are sent to an acetone circulation step comprising the separation system 20 where acetone is fractionated and fed back to the methanol removing device 1a set ahead of the reaction step.

### Method (iii):

This method comprises a crystallization step in which a crystalline adduct of bisphenol A and phenol is formed and slurried, a solid/liquid separation step in which the obtained slurry is separated into the crystalline adduct of bisphenol A and phenol and a liquid portion, and a step for separating the low-boiling point materials including unreacted acetone from the liquid portion obtained in the solid/liquid separation step.

Thus, in this method, the reaction mixture obtained in the reaction step is supplied directly to the crystallizer 5 (the supply line being not shown), and crystallization is carried out by adding, if necessary, water and acetone. In the crystallization operation, the bisphenol A concentration is set at usually 20 to 50% by weight, the water concentration at usually 1 to 10% by weight, and the acetone concentration at usually 0.5 to 5% by weight. The crystallization method is not specifically defined, but it is preferable to perform crystallization by cooling the crystallization materials by a heat exchanger. It is also possible to effect cooling by incorporating depressurization evaporation used in the method (ii). The crystallization materials having temperature of 70 to 140°C are cooled down to usually 35 to 60°C, whereby the crystalline adduct is crystallized and slurried. The obtained slurry is separated into solid and liquid by the solid/liquid separator 6.

The crystallization mother liquor from the solid/liquid separator 6 or a mixture of the mother liquor and the rinse waste from the solid/liquid separator 6 is transferred to the low-boiling point materials separating step. In case where crystallization is carried out by incorporating depressurization evaporation used in the method (ii), the evaporated low-boiling point materials are also transferred to the low-boiling point materials separating step.

In the low-boiling point materials separating step, usually a distillation column is used and the low-boiling point materials including unreacted acetone, water and phenol are separated from the column top by a method such as vacuum distillation. Vacuum distillation is carried out usually at a temperature of 50 to 150°C under a pressure of 50 to 300 mmHg. The separated low-boiling point materials including unreacted acetone, water and phenol are transferred to the acetone circulation step comprising the separation system 20 where acetone is fractionated and fed back to the methanol removing device 1a set ahead of the reaction step.

In case where one of the above methods (i) to (iii) is used, there exist in the low-boiling point component the alkylmercaptans produced with catalyst poisoned by methanol existing in a small quantity in the reaction mixture. It is therefore preferable to provide means for removing and discharging alkylmercaptans in the stream gas in separating the low-boiling point component. The alkylmercaptans removing method is not specifically defined; it is possible to employ various methods such as washing method, oxidation (ozonization) method, adsorption method, treatment with organisms, combustion, photocatalytic deodorization, plasma deodorization and deodorant spray. In case where the alkylmercaptan is dimethyl sulfide or the like and the washing method is used, the alkylmercaptan is oxidized into an acid substance such as sulfuric acid by using sodium hypochlorite as oxidizer, and then pH of the obtained substance is adjusted with sodium hydroxide or such. In the adsorption method, usually activated carbon is used for adsorption. This method is useful even for the treatment of the substances of a low concentration of less than several ppm, and its efficiency is enhanced if the activated carbon used for the adsorption treatment of odor is regenerated for reuse. In the combustion method, it is preferable to use a desulfurization system for the treatment of waste gas after combustion.

### Acetone circulation step:

In this step, unreacted acetone is separated and recovered from the low-boiling point component separated in the said separation step, and circulated back to the reaction step. Separation of unreacted acetone from the low-boiling point component is conducted in the separation system 20. Usually a multi-stage distillation column is used as the separation system 20, and water, acetone and phenol are separated. Methanol is contained as an impurity in the separated and recovered acetone. The separated and recovered acetone is passed through line 21 and mixed with fresh acetone, and the mixture is supplied to the reaction step. The separated and recovered phenol is refined together with fresh phenol by a refiner 20a, then stored in a rinse phenol storage tank 22 and used for the rinse of the crystalline adduct described later.

### Crystallization step:

In case where the method (i) is used in the separating step, the crystallization step is carried on with the bottom product (liquid portion containing bisphenol A) left after separation of the low-boiling point materials. In this crystallization step, as in the case of the methods (ii) and (iii), the materials are cooled from 70-140°C to 35-60°C in the crystallizer 5, then the crystalline adduct is crystallized and slurried, and this slurry is separated into solid and liquid in the solid/liquid separator 6.

As the crystallizer 5 in the methods (i)-(iii), usually a crystallization tank having a plurality of outside coolers capable of switchover operation is used. It is possible to prevent the outside coolers from freezing by providing a plurality of such outside coolers and operating them in turn. Since the crystallization characteristics in the crystallization tank change on switchover of the outside cooler, the amount of the mother liquor separated in the solid/liquid separator 6 fluctuates correspondingly. In order to prevent fluctuation of the amount of the mother liquor supplied from line 15 to line 1, usually part of the mother liquor is by-passed to the downstream side of the reactor 2 through line 16. In case of using the method (ii) while using a crystallization tank equipped with a jacket type cooler as the crystallizer 5, it is preferable to use a tank provided with a scraper for removing the scale deposited on the heat transfer surface.

### Bisphenol A recovery step:

The crystalline adduct recovered in the solid/liquid separator 6 after the separation step using any one of the above-described methods is re-dissolved in phenol in a re-dissolver 7, then recrystallized by a recrystallizer 8 to increase purity, separated into solid and liquid by the solid/liquid separation and rinse system 9, rinsed by rinse phenol, and then transferred to a crystalline adduct decomposition system 11.

The best part of the liquid portion separated by the solid/liquid separation and rinse system 9 and the rinse waste are used as phenol for re-dissolving the crystalline adduct in the re-dissolver 7 and as the rinse supplied to the solid/liquid separator 6. Part of the liquid portion separated by the solid/liquid separation and rinse system 9 is circulated to the tank 14 together with the circulating mother liquor.

In the crystalline adduct decomposing device 11, usually the crystalline adduct is heated to 100 to 160°C and thereby dissolved, and most of phenol is evaporated away from the obtained adduct solution. In the refiner 12, usually the residual phenol is removed by suitable means such as steam stripping to obtain refined bisphenol A. This method is described in, for instance, Japanese Patent Application Laid-Open (KOKAI) Nos. 2-28126 and 63-132850. Mother liquor circulation step:

The liquid portion (mother liquor) separated by the solid/liquid separator 6 is circulated to the upstream side and the downstream side of the reaction step through lines 13, 15 and 16.

The composition of the mother liquor obtained from the solid/liquid separator 6 is usually 65 to 85 wt% phenol, 10 to 20 wt% bisphenol A and 5 to 15 wt% by-products such as 2,4'-isomer. Thus the mother liquor contains impurities such as 2,4'-isomer at a high percentage. In order to prevent such impurities in the circulation system from accumulating, preferably the whole amount of the mother liquor obtained from the solid/liquid separator 6 is not circulated, but part of the mother liquor is taken out and subjected to an impurities removing treatment to elevate purity, and then it is supplied to the circulation system.

As the impurities removing treatment, a method is usually used in which part of the mother liquor from line 13 is fed into the impurities removing device 13a where a decomposing catalyst such as sodium hydroxide is added to the fed mother liquor and the mixture is distilled, recovering phenol, bisphenol A and isopropyl phenol as the column top products, with the recovered products being returned to line 13. The bottom products are discharged out of the system.

The ratio of the mother liquor separated and fed into the impurities removing device 13a from line 13 is usually 4 to 15% by weight. Owing to this impurities removing device 13a, the composition of the mother liquor circulated to the tank 14 from line 13 is stabilized to remain substantially in the above-defined range.

A small quantity of the cation exchange resin residue derived from the reaction step is contained in part of the fed mother liquor, so that it is preferable to provide means for removing the cation exchange resin residue from the mother liquor and then again returning it to line 13.

The mother liquor from line 13 is supplied by way of tank 14 and, through lines 15 and 16 to lines 1 and 3, respectively. The mother liquor pumped out from the tank 14 is controlled by a valve provided on line 15 to keep the flow rate in line 16 constant.

The operation of the pump (not shown) for delivering the mother liquor from the tank 14 to line 15 is controlled so that the molar ratio of the amount of phenol to the total (a + b) of the amount of fresh acetone (a) supplied to line 1 and the amount of recovered acetone (b) supplied through line 21 (phenol/acetone) will maintain constant in the range shown above.

Next, the characteristic features of the first aspect of the present invention are explained. The first aspect of the present invention is characterized in that the lower alcohol concentration in the whole amount of acetone supplied to the reaction step is adjusted to be not more than 100 ppm by weight, preferably 20 to 100 ppm, more preferably 30 to 80 ppm. "Lower alcohols" referred to herein designate the alcohols with a carbon number of 1 to 8, a typical example of which is methanol. In the following, the cases involving methanol as lower alcohol are explained. (The same holds true with the description of the characteristic features of the second and third aspects given later.)

The methanol concentration in fresh acetone is usually 50 to 400 ppm by weight (hereinafter referred to simply as ppm), and the methanol concentration in recovered acetone is about 70 to 700 ppm. The ratio of the amount of recovered acetone (b) to the amount of fresh acetone (a) (b/a) is usually about 0.05 to 0.2. Therefore, in case where no methanol removing treatment is conducted, the methanol concentration in the mixture of fresh acetone and recovered acetone is usually about 50 to 500 ppm. However, even if the methanol concentration in fresh acetone is less than 100 ppm, the methanol concentration in recovered acetone rises up while the reaction is carried on, causing the methanol concentration in the mixed acetone to exceed 100 ppm. Also, in case where the methanol concentration in fresh acetone is over 100 ppm, there is a possibility that the methanol concentration in the mixed acetone would exceed 100 ppm from the very beginning.

In either case, methanol in acetone is removed at the stage where the methanol concentration exceeded 100 ppm. In case where the methanol concentration in fresh acetone is below 100 ppm, there may be used, for example, a method in which after removing methanol in recovered acetone, the latter is mixed with fresh acetone, or a method in which the ratio of the amount of recovered acetone (b) to the amount of fresh acetone (a) (b/a) is adjusted. When the methanol concentration in fresh acetone exceeds 100 ppm, methanol in the mixed acetone is removed. In either method, it is tried to keep below 100 ppm the methanol concentration in the whole acetone supplied to the reaction step. The methanol concentration in acetone can be determined by gas chromatographic analysis. The method for removing methanol in acetone is discussed later.

Now, the characteristic features of the second aspect of the present invention are described. The second aspect of the present invention is characterized in that the lower alcohol concentration in the reaction solution discharged from the reaction step is adjusted to stay at not more than 30 ppm, preferably not more than 20 ppm, more preferably not more than 10 ppm. In other words, since the lower alcohols do not participate in the reaction, the concentration of lower alcohols such as methanol existing as impurities in the mixture of the materials including phenol, fresh acetone and recovered acetone supplied to the reaction step is kept to be not more than 30 ppm.

In the case of an acid ion exchange resin partially neutralized with a sulfur-containing amine compound, in addition to formation of a sulfide by the reaction of methanol and the promotor mercapto groups, the reaction is allowed to proceed further to form sulfonium cations to reduce the activity at the acid site. In this case, there are generated the odorant material alkylmercaptans such as methylmercaptan and dimethyl sulfide (decomposition elimination products from the promoter) concomitant with poisoning catalyst. Therefore, in the case of an acid ion exchange resin partially neutralized with a sulfur-containing amine compound, it is particularly important to lower the concentration of methanol supplied to the reactor. Methanol concentration in the reaction solution can be determined by gas chromatographic analysis.

For reducing the methanol concentration in the said mixture of materials supplied to the reactor, there can be used, for example, the below-described method in which the methanol concentration in acetone is reduced.

The characteristic points of the third aspect of the present invention are described here. The third aspect of the present invention is characterized in that in the whole amount of acetone supplied to the reaction step, at least the acetone recovered from the reaction step is refined before supplied to the reaction step to remove the lower alcohols contained as impurities in that acetone. The acetone from which the lower alcohols such as methanol are to be removed may be either recovered acetone or a mixture of fresh acetone and recovered acetone. In case where the methanol concentration in fresh acetone is low, it is expedient to try to remove methanol in recovered acetone. In case where the methanol concentration in fresh acetone is high, it is expedient to remove methanol in the mixture of fresh acetone and recovered acetone.

Removal of methanol in acetone is carried out using a methanol removing device 1a so that the concentration of methanol existing as an impurity in acetone supplied as a reaction material will be reduced to usually below 100 ppm, preferably to a level of 20 to 100 ppm, more preferably 30 to 80 ppm.

Exemplary of the methanol removal method using methanol removing device 1a is the method disclosed in Japanese Patent Application Laid-Open (KOKAI) No. 9-278703. Preferably, a distillation column is used, and methanol-containing acetone is distilled in this column with the operating pressure adjusted to stay usually not less than 200 kPa, preferably in the range of 200 to 1,100 kPa, more preferably 300 to 600 kPa (as for instance described in Japanese Patent Application Laid-Open (KOKAI) No. 9-278703). As the distillation column, a packed column or a plate column with a theoretical plate number of usually not less than 20, preferably 25 to 50, is used. The column top temperature and bottom temperature are selected in accordance with the column top operating pressure. In a preferred embodiment, acetone containing a small quantity of methanol is supplied to the middle portion of distillation column and the column is operated under the pressure specified above to fractionate methanol-rich acetone from the column top while obtaining the acetone solution markedly reduced in methanol concentration from the bottom. This bottom product is supplied as a reaction material through line 1.

### EXAMPLES

The present invention will be described in further detail with reference to the examples thereof, but it should be understood that the present invention is not limited to these examples but can be embodied otherwise as well without departing from the scope of the invention. In the following description, all percents (%) are by weight unless otherwise noted. The quantity of bisphenol A and methanol concentration were determined by gas chromatographic analysis using "GC-17A" mfd. by Shimadzu Corp. and an FID detector.

### Example 1

Bisphenol A was produced by a pilot plant having a flow pattern shown in FIG. 1. The methanol concentration in fresh acetone was 300 ppm. A distillation column having an ordered packing ("MC Pack MC350S" produced by Ryoka Forward Co., Ltd.) with a theoretical plate number of 35 was used as methanol removing device 1a, and under a pressure of 410 kPa, a mixture of fresh acetone and recovered acetone from line 21 were supplied from the 25th plate from the top.

Used as the catalyst in the reactor 2 was a sulfonic acid type cation exchange resin (AMBERLYST 31, produced by Rohm & Haas Company) with 20 mol% of sulfonic groups being partially neutralized with 2-(4-pyridyl)ethanethiol. The resin was packed to a volume of 3.4 m³. The new feed of acetone was 60 kg/hr. The feed of recovered acetone from line 21 was 10 kg/hr on the average. The flow rate in line 16 was adjusted so that the feed of the mother liquor from line 15 to line 1 would become constant at 1,608.8 kg/hr.

The bottom temperature of the distillation column 4 was set at 135°C and the pressure at 100 mmHg. Used as crystallizer 5 was a crystallization tank provided with three outside coolers, and the crystallization step was carried out by suspending the operation of one of the three outside coolers in turn. Temperature of the crystallization tank varied in the range between 49 and 51°C.

As a result, the methanol concentration in the recovered acetone from line 21 was 515 ppm on the average. The acetone conversion in the catalyst had dropped gradually from 99.5% at the start of the reaction. The amount of recovered acetone from line 21 also increased gradually during this period, but the methanol concentration in acetone after the treatment by methanol removing device 1a was maintained at 90 to 109 ppm. The methanol concentration in the reaction solution after mixing with the mother liquor supplied to line 1 was kept at 3 to 4 ppm. The acetone conversion after the elapse of 12 months was 81%. It was possible to continue the operation over a period of 12 months with no need of changing the catalyst in the reactor 2.

### Comparative Example 1

The same operations as in Example 1 were carried out except that the methanol removing device 1a was set on line 21, the recovered acetone from line 21 alone was subjected to the distillation treatment under normal pressure by using the methanol removing device 1a, the thus treated recovered acetone was mixed with fresh acetone and supplied to the reactor 2 from line 1, and the supply of the mother liquor from line 15 to line 1 was changed to 500 kg/hr. The methanol concentration in recovered acetone after the methanol removing treatment dropped only to the level of 900-1,000 ppm. The acetone conversion of the catalyst lowered gradually from 99.9% at the start of the reaction, with the amount of recovered acetone also increasing gradually, and the methanol concentration in the mixture of recovered acetone and the new feed of acetone increased to 300-325 ppm, higher than the methanol concentration in fresh acetone. Also, the methanol concentration in the reaction solution after mixing with the mother liquor increased to 32-34 ppm. In approximately 6 months after start of the operation, the acetone loading dropped to 69% and the catalyst in the reactor 2 deteriorated, requiring change of the catalyst with new one. It was thus impossible to carry on the operation for a long time.

### Description of Reference Numerals:

- 1a:: methanol removing device
- 2:: reactor
- 4:: distillation column
- 5:: crystallizer
- 6:: solid/liquid separator
- 7:: re-dissolver
- 8:: recrystallizer
- 9:: solid/liquid separation and rinse system
- 11:: crystalline adduct decomposing device
- 12:: refiner
- 13a:: impurities removing system
- 14:: tank
- 20:: separation system
- 20a:: refiner
- 22:: phenol storage tank

## Claims

1. A process for producing bisphenol A by reacting the raw materials acetone and phenol in the presence of a catalyst,
the lower alcohol concentration in the whole amount of acetone supplied to the reaction step being adjusted to be not more than 100 ppm by weight, when in supplying to the reaction step the said raw materials and the acetone recovered from the reaction step.

2. A process for producing bisphenol A by reacting the raw materials acetone and phenol in the presence of a catalyst,
the lower alcohol concentration in the reaction solution discharged from the reaction step being adjusted to be not more than 30 ppm by weight, when in supplying to the reaction step the said raw materials and those recovered from the reaction step.

3. A process for producing bisphenol A by reacting the raw materials acetone and phenol in the presence of a catalyst, at least the acetone recovered from the reaction step in the whole supply of acetone to the reaction step being refined before supplied to the reaction step to remove the lower alcohols contained as impurities, when in supplying to the reaction step the said raw materials and the acetone recovered from the reaction step.

4. The process according to Claim 3, wherein fresh acetone and the acetone recovered from the reaction step are mixed, and the mixture is refined before supplied to the reaction step to remove the lower alcohols contained as impurities.

5. The process according to Claim 3, wherein the acetone recovered from the reaction step is refined to remove the lower alcohols contained as impurities, with the concentration of the lower alcohols contained in the recovered acetone being made less than the concentration of the lower alcohols contained in the fresh acetone.

6. The process according to any one of Claims 3 to 5, wherein the treatment for removing the lower alcohols is distillation treatment carried out under a pressure not less than 200 kPa.

7. The process for producing bisphenol A according to Claim 6, wherein the distillation treatment is carried out under a pressure of 200 to 1,100 kPa.

8. The process according to any one of Claims 1 to 7, wherein the catalyst used in the reaction step is an acid ion exchange resin partially neutralized with a sulfur-containing amine compound.
